# EUROPEAN PATENT SPECIFICATION

(11) **EP 0 406 407 B1**
(45) Date of publication and mention of the grant of the patent: **19.07.1995**
(21) Application number: 90902866.4
(22) Date of filing: 18.01.1990
(51) Int. Cl.: A61M 5/44

(54) **RAPID INFUSION DEVICE**
SCHNELLINFUSIONSVORRICHTUNG
DISPOSITIF DE PERFUSION RAPIDE

(30) Priority: 19.01.1989 US 299099
(43) Date of publication of application: 09.01.1991
(73) Proprietor: STEPHENS, Harry William jr, Allentown, PA 18103 (US); MANLEY, Norman James, Allentown, PA 18105 (US); MONTESANO, Ralph Michael, Macungie, PA 18062 (US)
(72) Inventor: STEPHENS, Harry William jr, Allentown, PA 18103 (US); MANLEY, Norman James, Allentown, PA 18105 (US); MONTESANO, Ralph Michael, Macungie, PA 18062 (US)
(74) Representative: Held, Martin
(86) International application number: US9000176
(87) International publication number: WO9007947

(56) References cited:
- EP-A- 0 174 420
- EP-A- 0 234 713
- EP-A- 0 292 399
- FR-A- 2 188 392
- FR-A- 2 513 884
- US-A- 4 115 277
- US-A- 4 196 729
- US-A- 4 368 118
- US-A- 4 678 460
- US-A- 4 747 826

## Description

This invention relates to a device as defined in the preamble of claim 1 for the rapid infusion of circulatory supportive fluids such as blood into a patient. The device comprises both durable or permanent components and disposable sub-components. The disposable sub-components include various components which physically come into contact with the fluid being infused. The principal advantage achieved from this arrangement resides in the fact that after each use, the disposable components are destroyed and replaced with new sterile components to preclude contamination from patient to patient as well as from medical personnel to patient.

### BACKGROUND OF THE INVENTION

During surgery or in the emergency room, it is frequently necessary to infuse blood rapidly into a patient, particularly when massive blood losses have occurred. Patients having inadequate blood volume can suffer serious consequences.

There are many situations where large amounts of blood can be lost in a very short period of time, for example, in cases of serious automobile accidents, gun shot wounds in critical areas of the body, and a variety of major surgery including cancer surgery and heart and liver transplants.

In the past, the replacement of large amounts of blood loss has been a major problem to the surgical teams attending a suffering patient. A common method of rapid infusion includes the use of a plurality of infusion sites simultaneously. Infusion bags or bags of stored banked blood are interconnected by intravenous tubing. Frequently, a plurality of medical personnel are required to oversee the various infusion sites and to personally ensure the flow of blood from the blood bags or monitor the pumps which infuse the blood to the patient.

It is also well known that the temperature of the blood to be infused into a patient is a critical item which must be monitored closely. In this regard, to maintain the infused blood at the desired temperature, various arrangements of heating coils have been strategically placed around the infusion, thus increasing the complexity of arrangement and requiring additional personnel to ensure successful operation. FR-A-2 188 392 discloses a device of the kind as defined in the preamble of claim 1 with which micro-wave energy is used for heating the blood to be infused. However, applying of micro-waves to the blood may be detrimental to the blood-cells.

It can readily be seen from the foregoing, that any apparatus which would satisfy the various requirements of rapid infusion while at the same time reducing the number of medical and/or technical personnel required to monitor the equipment would be a much desired improvement over presently known systems and practices used in such critical life threatening situations.

### SUMMARY OF THE INVENTION

With knowledge of the shortcomings of present day blood infusion apparatuses noted above, applicants have been motivated to develop the rapid infusion device herein disclosed and claimed in claim 1 of the instant application. The rapid infusion device, referred to hereinafter as RID, is composed of two systems. One system includes durable equipment, i.e., equipment which can be used over and over again, such as the roller pump and its related controls, a permanently mounted heating element and its related controls, and mounting bracket structure for attaching the heating element to the housing of the roller pump. The other systems, include the disposable components of the RID such as the blood reservoir. According to various embodiments, there are also included a three stage filter insert, aluminum-telfa interior wall of the heat exchange unit, two-way valves and a spring loaded pressure control valve. Additionally, all volume pathways are cast as a part of a disposable unit housing. A disposable length, of for example one quarter inch PVC tubing, is also provided to connect the filter-reservoir to the heating unit.

The disclosed Rapid Infusion Device is a mechanical pumping system for rapidly delivering filtered, bubble-free, warmed blood to a patient suffering from acute hypovolema. The principle of operation of the disclosed invention is one of overcoming resistance mechanically. The flow of fluids is totally regulated by the resistance encountered at the smallest tubing orifice along the infusion pathway. The word volume is used to describe or refer to the fluid delivered by the device as any desired fluid other than blood may also be delivered by the device.

The RID provides an apparatus for the rapid infusion of volume being pumped. Additionally, the RID provides a means of de-aerating the volume and also a heating means for maintaining the volume at the desired temperature. The pump utilized is a variable speed unit so that the amount of volume being pumped in can be increased merely by increasing the rpm of the pumping unit. The reservoir contains plural stages of filters and is provided with a plurality of inlets whereby volume from multiple sources can be fed into the reservoir to satisfy any high demand requirements of a patient. As set forth above, the RID comprises a permanent system and a disposable system. The disposable system includes all the components with which the volume comes into direct contact. The disposable aspect of the invention provides an extra measure of protection against contamination to a subsequent patient.

With the foregoing in mind, it is a primary object of the present invention to provide an apparatus which is capable of high volume pumping.

Another object of the invention is to provide an apparatus having a permanent system and a disposable system.

A further object of the invention is to provide a RID in which the disposable portion includes all components which come into direct contact with the volume being infused.

Yet another object of the invention is to provide an apparatus which de-aerates and heats the volume being infused.

Still another object of the invention is to provide an apparatus which is compact, multi-functional, and capable of operation by a single person.

A further object of the invention is to provide a RID which includes a multi-filter arrangement for cleansing the volume prior to infusion.

Another object of the invention is to provide a RID which also includes a pressure control valve for regulating system pressure and, more specifically, infusion pressure to the patient.

Other objects and advantages of the instant invention, in addition to those set forth above, will become more apparent from the ensuing detailed description considered in conjunction with reference to the accompanying drawings which form a part of this specification wherein like reference characters designate corresponding parts in the several views.

### BRIEF DESCRIPTION OF THE DRAWINGS

Referring briefly to the drawings, the reader will readily appreciate that:
Fig. 1 is a front elevational view of the rapid infusion device;
Fig. 2 is also a front elevational view of the rapid infusion device in which all of the disposable components are shown in broken lines;
Fig. 3 is a front elevational view illustrating the durable or permanent non-disposable components of the device;
Fig. 4 is a front elevational view of the durable heating element along with the disposable components per se;
Fig. 5 is a sectional view of the filter/reservoir taken along the line 5-5 of Fig. 4;
Fig. 6 is an elevational view of the filter frame per se;
Fig. 7 is a sectional of the filter illustrating the plurality of layers;
Fig. 8 is an illustration of the disposable heating unit assembled with the durable or permanent heating element;
Fig. 9 is an elevational view similar to Fig. 8, but with the disposable components illustrated in broken lines;
Fig. 10 is an enlarged detail view of the spring loaded pressure control valve;
Fig. 11 illustrates the flow of volume in the purge mode of operation;
Fig. 12 illustrates the flow of volume during the by-pass mode of operation;
Fig. 13 illustrates the normal mode of operation of the RID with volume flow to patient; and
Fig. 14 illustrates the flow of volume to patient with the pressure control valve open.

### DETAILED DESCRIPTION OF THE DRAWINGS

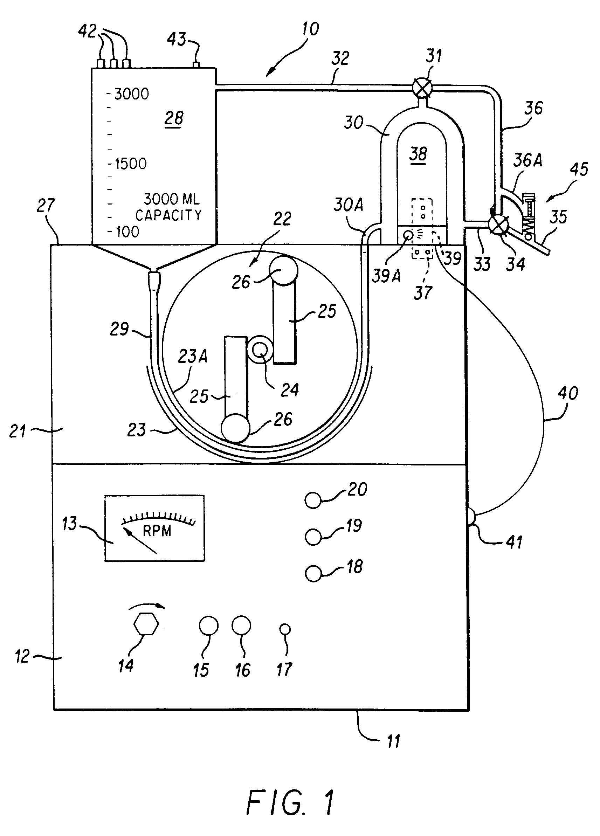

Referring now in detail to Fig. 1, the reader will see from the front elevational view of the disclosed invention that the rapid infusion device generally indicated by reference numeral 10 comprises a base portion 11 which houses a control panel 12 having a tachometer 13, rpm control knob 14, AC power fuse 15, DC power fuse 16, and on/off switch 17. On the upper right portion of control panel 12 are located AC/DC selector switch 18, stop/forward switch 19 for a motor (not shown) for driving roller pump 22. Above stop/forward switch 19 is located the reverse switch 20 for reversing the rotation of roller pump 22.

Mounted on top of control panel 12 is pump housing 21 which serves as the mounting support for roller pump 22 and its drive motor (not shown). Roller pump 22 comprises a pair of walls 23, 23A forming a raceway, which tubing 29 is inserted into and supported thereby. A pair of roller support arms 25 extend from shaft 24, each of which supports a roller 26 at the distal end thereof for engagement with tubing 29. Each of the support arms 25 extend in opposite directions with respect to each other from shaft 24.

Mounted on upper surface 27 of pump housing 21 are the various components of the disposable unit including filter/reservoir 28, heat exchange component 30 and associated tubing line 29 leading from filter/reservoir 28 to roller pump 22 which discharges pumped volume to heat exchange component 30 which is in the form of an inverted U-shaped member. The upper portion of heat exchange component 30 is interconnected with two-way purge by-pass valve 31 which connects with purge by-pass line 32 back to filter/reservoir 28. The right leg of heat exchange component 30 includes an outlet line 33 which connects with the by-pass to patient two-way valve 34. The left leg of heat exchange component 30 includes an inlet stem portion 30A for connection with line 29. Two-way valve 34 can be set to direct pumped volume to either patient line 35 or purge by-pass line 36 back to filter/reservoir 28.

Bracket 37, which is shown in dashed lines, serves as the mounting bracket for permanent heating element 38. Thermostatic control of permanent heating element 38 is provided by knob 39 for controlling the temperature of the volume. Extending from the base of permanent heating element 38 is electrical cord 40 provided with plug 41 which is plugged into control panel 12 and thereby supplies electrical current to permanent heating element 38. As indicated above, the device 10 is provided with AC and DC controls in the event that there is a power failure or the device 10 is to be used in an area where AC power is not available.

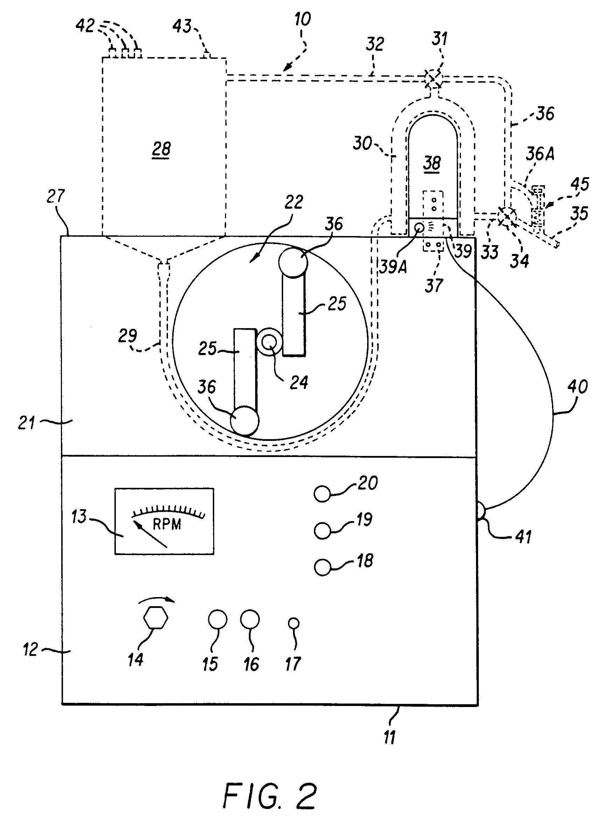

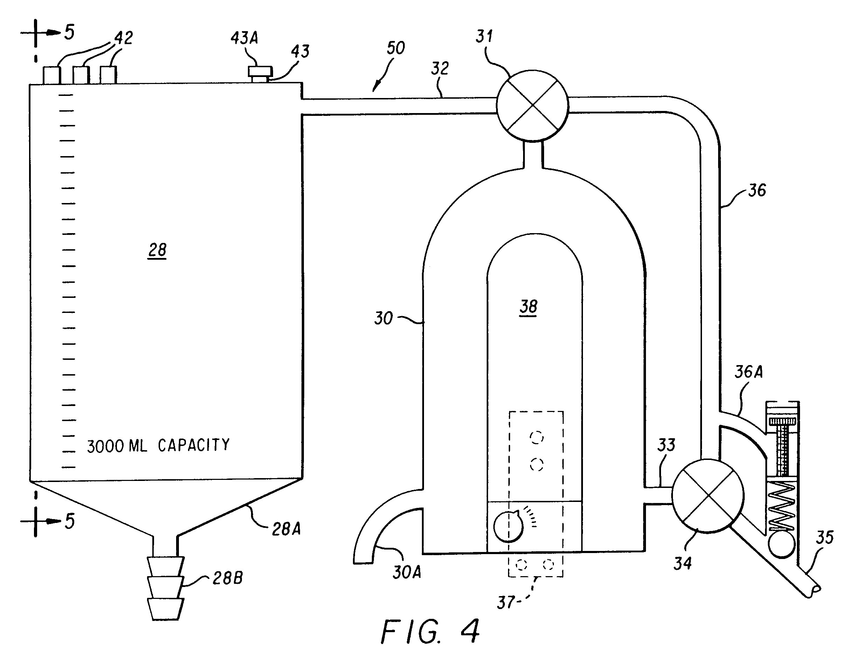

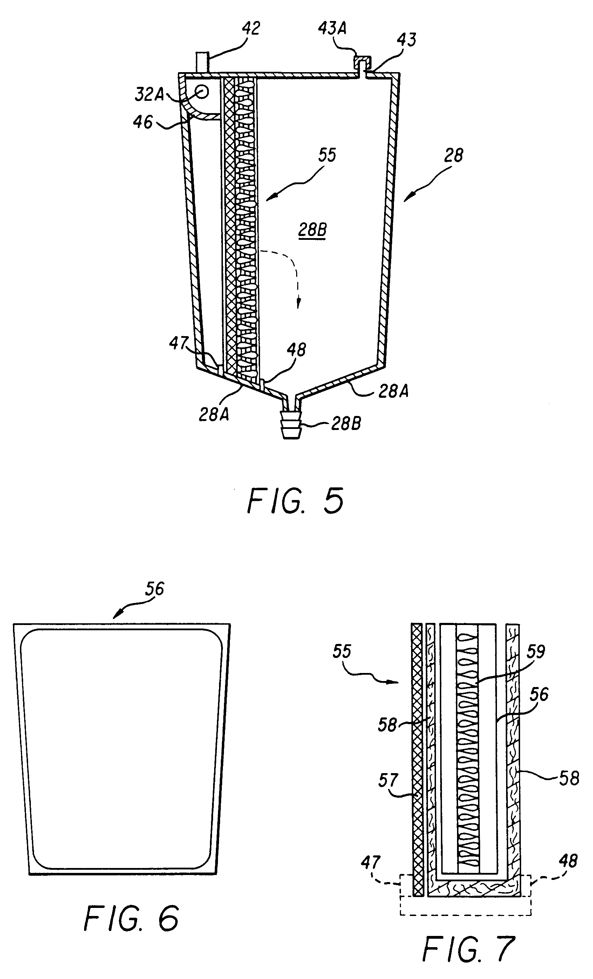

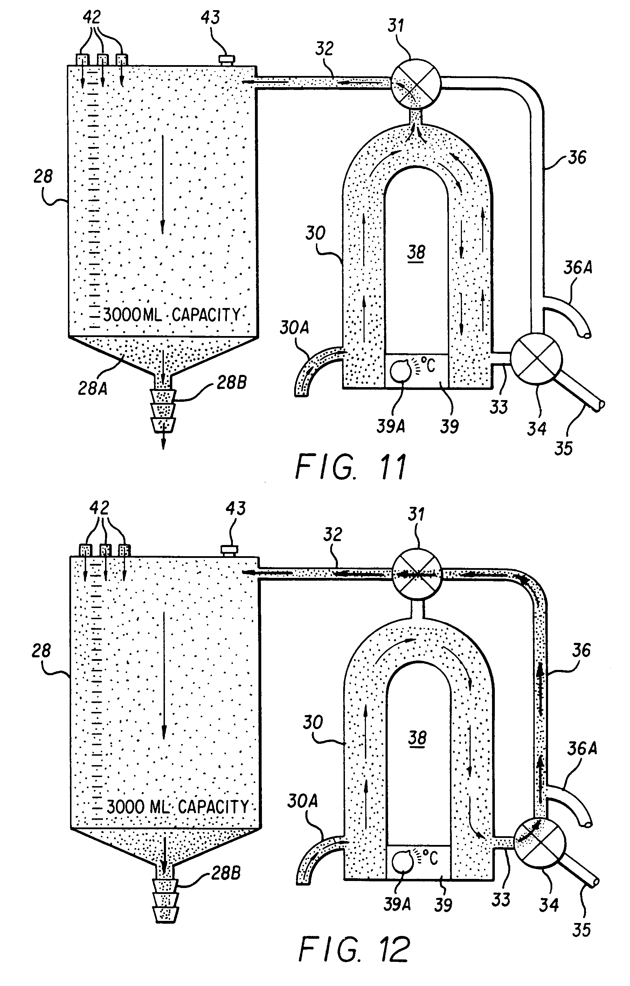

Referring now to Fig. 2, the reader can readily see basically showing the same structure illustrated in Fig. 1, but with the entire disposable system shown in dashed lines in Fig. 2. As illustrated, filter/reservoir 28 is provided with a plurality of fluid inlet ports 42 to enable rapid replenishment of the volume in filter/reservoir 28. Additionally, air vent 43 is shown at the upper right corner of filter/reservoir 28 with vent cap removed. Fig. 2 provides a complete view of all the disposable components. The disposable components illustrated provide pathways for volume in the various operating modes of the system. The disposable unit provides access to the system through its three inlet ports 42. The volume to be pumped through the system may be pumped, poured, or drained into the filter/reservoir 28 through ports 42. Temporary storage, filtration, heating and the direction of volume flow is controlled by the disposable unit. System pressure is regulated in the unit by the spirng loaded pressure control valve 45. De-aerating of volume is accomplished by removing the cap on air vent 43 to allow the passage of air in and out of the system and the positioning of the purge by-pass two-way valve 31. When venting is not desired, air vent 43 is closed by cap 43A thereover as illustrated in Figs. 4 and 5. Flow to the patient and alternative by-pass route for volume is achieved in the unit by setting the by-pass-to-patient two-way valve 34 in the desired position provided for in Figs. 11-14.

The disposable unit combines the filter/reservoir 28 with its filtration function, heat exchange component 30, two directional control valves 31 and 34 and the system pressure regulating control valve 45 into one unit. This unit is disposable as it comes in contact with volume and is considered potentially contaminated and not to be re-used. All parts of the system coming in contact with either volume or patient are disposable, including the PVC connecting line 29 between the filter/reservoir 28 and heater unit 30 and the catheter (not shown) connected to line 35 to the patient for infusing the patient.

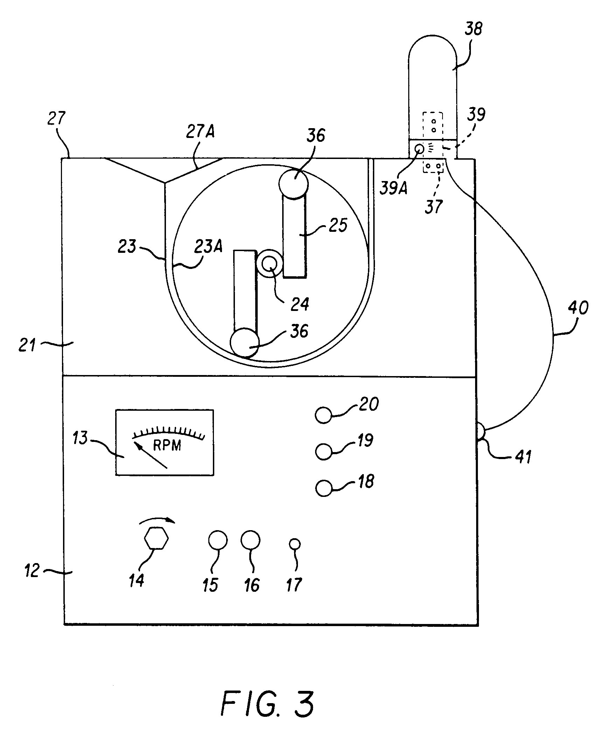

Referring now to Fig. 3, the reader can readily see the durable or permanent non-disposable components of RID 10 with the relationship of the permanent components such as heating element 38, its supporting bracket 37 which serves as its mount, and thermostat 39 with its control knob 39A. Additionally, conical recess 27A, which receives and supports the conical lowermost portion 28A of the filter/reservoir 28 when inserted therein, is readily seen. Omitted from Fig. 3 is tubing line 29 which is normally disposed in the raceway formed by walls 23 and 23A since tubing line 29 is removed as with the parts of the disposable unit.

Referring now to Fig. 4, there is illustrated a complete showing of the disposable unit 50 of the RID 10 and its relationship to permanent heater element 38. The only two disposable items not illustrated here are the piece of PVC tubing 29 which interconnects fitting 28B of filter/reservoir 28 with stem 30A of heater unit 30 and the catheter (not shown) which connects with line 35 to the patient.

Disposable unit 50 shown in Fig. 4 in operation provides filtration of volume in filter/reservoir 28, acts as a vessel to provide temporary storage volume in the reservoir 28, and provides a controlled pathway for volume to flow, when valves 31 and 34 are set in their desired positions. Heat exchange is also accomplished in disposable unit 50 by way of the relationship of heat exchange component 30 with permanent heating element 38.

The housing of the disposable unit 50 is made of plastic, molded in two halves, filters, valves and the aluminum-telfa interior wall of the heating unit 30, are installed and the halves are then joined, adhered and sealed for liquid integrity. The disposable unit 50 is designed for a single use and is destroyed thereafter. Similarly, all other pathways coming into direct contact with volume are disposed of and/or destroyed, a safety feature to avoid possible contaminaton of the disposable unit 50 and other volume pathways. Other volume pathways include all PVC lines 29 and 35 used in the device. Although permanent heating element 38 is illustrated in Fig. 4 to show its relationship with the disposable unit 50, it is a durable permanent part of RID 10 and is mounted on pump housing 21 via bracket 37.

Referring now to Fig. 5, there is illustrated a cross-sectional view taken along the line 5-5 of Fig. 4 showing one of the three inlet ports 42 since the other two inlet ports 42 are hidden by first inlet port 42. There is also shown the inlet port 32A for purge by-pass line 32. Baffle 46 is located in the upper left interior corner of filter/reservoir 28 and extends the full width thereof. Filter 55 is shown standing vertically in filter/reservoir 28. At the left conical portion 28A, a pair flanges 47 and 48 project upwardly therefrom permitting filter 55 to rest therebetween and retained thereat on the left of conical portion 28A.

Filter/reservoir 28 provides another safety feature with the added function of storing volume to be infused into the patient. In a preferred embodiment of the invention, the capacity of the vessel is 3000 ml. The baffle 46 is molded into the filter/reservoir 28 housing during manufacturing and functions to create a vortex minimizing any effect which flow may have on the volume to be infused. The baffle 46 directs the incoming volume into the coarse side of the filter 55. The air vent 43 in filter/reservor 28 is operated by loosening screw cap 43A on vent 43 to allow air to enter and leave RID 10. By-pass inlet port 32A allows for the return of volume to filter/reservoir 28 when the device is being purged of air, operated in the by-pass mode and when pressure to the patient has become great enough to open the spring pressure control valve 45.

Fig. 6 shows details of the filter frame 56 per se which has the purpose of holding the Cottonow "3" material 59 which is part of the three layered second stage of filter 55.

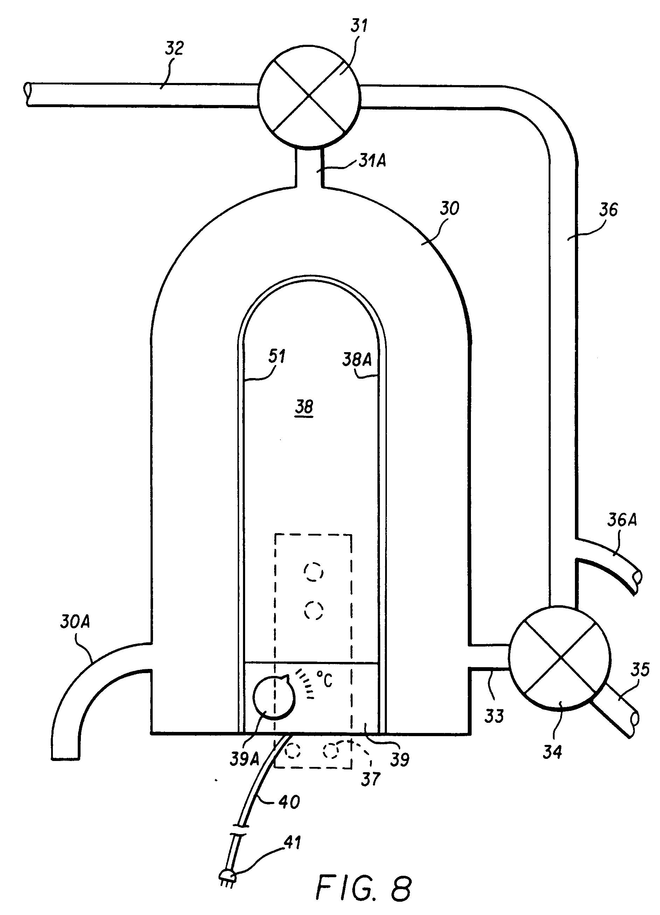

Referring now to Fig. 7, there is shown a two stage filtration system including a first stage with a layer of coarse sponge plastic material 57 which is positioned on the fill side of the filter/reservoir 28 and functions to remove material in the volume of over 100 to 150 microns in size. The coarse filter material 57 is compressed against the next three layers of filter material in which fine filtration takes place.

In the second stage, fine filtration functions to remove any remaining impurities in the volume of 27 to 40 microns in size. Fine filtration of volume is achieved by passage through three layers of filter material, including a first layer of nylon mesh 58, followed by Cottonow "3" material 59, and a second layer of nylon mesh 58. As fine filtration takes place, volume is passed into front reservoir chamber 28B for circulation in the system. The filter 55 is assembled by inserting Cottonow filter material 59 in the center of the frame 56. A fine nylon mesh "sock" 58 fused into two sides is slipped over the frame 56 containing the Cottonow material 59, forming three layers. The coarse layer 57 of filter material is then applied to the frame 56 to complete the wafer effect. Coarse layer 57 is made of plastic sponge material woven in a matrix to prevent particles in excess of 150 microns from passing through it. After the frame 56 is assembled with filter media, it is installed and sealed in the filter seat formed by seat flanges 47 and 48.

Referring now to Fig. 8, heat exchange component 30 may be seen with its various connections 30A, 33, and 31A to the various pathways of the system. As illustrated, heat exchange component 30 includes a hollow interior to permit the passage of volume therethrough and is shaped in the form of an inverted-U. The inverted-U member is made of an aluminum alloy with the inner wall 51 coated with aluminum-telfa to assist in the conduction of heat from heating element 38 to heat exchange component 30 and thence to the volume flowing therethrough. The exterior aluminum wall 38A of the heating element 38 and the interior wall 51 of heat exchange component 30 are disposed in a male-female relationship.

Heat exchange component 30 receives its volume supply from roller pump 22 which has created the flow for the entire system. As volume flows through heat exchange component 30, heat exchange between volume and the walls 38A and 51 takes place and recirculated volume is heated to a pre-set temperature by the setting of thermostat control knob 39A. The heater element 38 is heated by 115 volts, AC current via grounded cord 40 plugged into an accessory outlet on the control panel 12. The heat exchange component 30 is considered to be another safety feature, as infusing volume to a patient under 37 degrees C., can cause the patient to go into a state of shock.

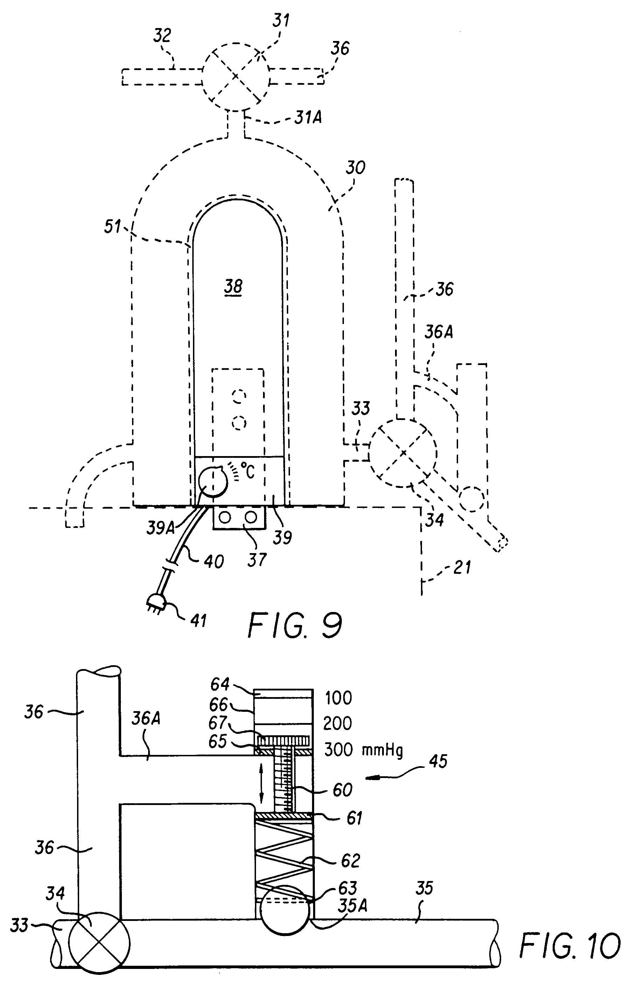

Turning now to Fig. 9, the heat exchange component 30 and the pump housing 21 may be seen in dashed lines. Heating element 38 has an upper arched portion which conforms to U-shaped configuration of heat exchange component 30. Thermostat 39 may be seen with control knob 39A for setting the desired temperature in Fig. 9. As pointed out earlier, heating element 38 and thermostat 39 are durable components of the RID 10.

Spring loaded pressure control valve 45, which may be seen in Fig. 10, provides another of the enumerated safety features of the RID 10. As shown, pressure control valve 45 comprises a threaded screw 60 which is threaded into stationary disc 65. Extending upwardly above valve 45 is a graduated cylindrical housing 66 with a removable cap 64 thereon to permit access to the screw head 67 which is provided with a slot (not shown) in its upper surface to receive a screwdriver to make the necessary adjustment to the valve 45. Attached to the lowermost end of screw 60 is a perforated base disc 61 which biases spring 62 into engagement with ball 63. Disc 61 is perforated to permit volume to flow therethrough when ball 63 is unseated from seat 35A.

The function of valve 45 is to regulate system pressure and, more specifically, infusion pressure to the patient. As can be seen, valve 45 includes a spring loaded, adjustable ball valve 63 which seats on seat 35A, with helical spring 62 having a known resiliency that can be adjusted with compression. When the screw 60 is advanced, compression on the spring 62 is increased and when withdrawn, decreased. The adjustable limits of the valve 45 are limited by the distance the adjusting screw 60 can travel in its housing 66. An adjustable range of 100 to 300 mm Hg is provided and access to the adjustment screw 60 is obtained through the access port when cap 64 is removed. The normal setting for valve 45 is in the range of 230-300 mm Hg, but may be changed depending on the body size of the patient or any other variable which may indicate that a change in pressure is desirable.

The pressure control valve 45 is positioned as near as possible to the patient to sense an overpressure condition. When such a condition exists, the valve 45 will open by unseating from seat 35A and volume will escape around the ball 63 through perforations in disc 61 and back through the system in the pathways 36A, 36, and 32. The illustrations of the valve 45 are not intended to be "as built" drawings, but rather to indicate its position and general construction characteristics.

Fig. 11 is the first of four figures depicting the flow of volume in four operating modes of the device. In Fig. 11, as in Figs. 12-14, the filter/reservoir 28 is filled from a source of volume. The volume to be pumped through the system may be supplied to the device from a variety of containers or methods to keep the filter/reservoir 28 at any desired level.

The objective of the mode of operation illustrated in Fig. 11 is to purge the system of air. The filter/reservoir outlet 28B is connected directly to the pump 22, and volume flows from the outlet port 28B through the line 29 fed through the pump. The outlet of line 29 from pump 22 is connected directly to inlet port 30A of the heat exchange component 30. Flow is developed by the action of roller pump 22 as the rollers 26 impinge on line 29 forcing volume therethrough. The two-way by-pass to patient valve 34 is closed to the patient and closed to the purge-by-pass line 36.

The purge-by-pass two-way valve 31 is opened to the filter/reservoir 28, and open to the air purge position, to allow the evacuation of air via line 32 and reservoir vent 43. The duration of this mode of operation is for a period sufficient to make the system air free.

This mode of operation is a further safety procedure and prevents the infusion of air to the patient.

Referring now to Fig. 12, there is shown the flow of volume during the by-pass mode. This mode of operation normally follows the system purge procedure illustrated in Fig. 11 and is also preliminary to the operation of the device, i.e., volume to patient.

The filter/reservoir 28 is filled from the volume source and flow follows its normal course from the roller pump 22 and into the inlet port 30A of heat exchange component 30. Heat exchange component 30 has been set at the desired temperature setting. Two-way by-pass to patient valve 34 is closed to the patient and opened to the filter/reservoir 28, and volume is continuously recirculated until the device is ready for use, i.e., until the volume has been heated to the desired temperature. This mode is another very desirable safety feature since it raises the temperature of refrigerated or ambient temperature volume to body temperature before it is infused to the patient thereby avoiding a shock effect to the patient which would be caused by sub-body temperature volume.

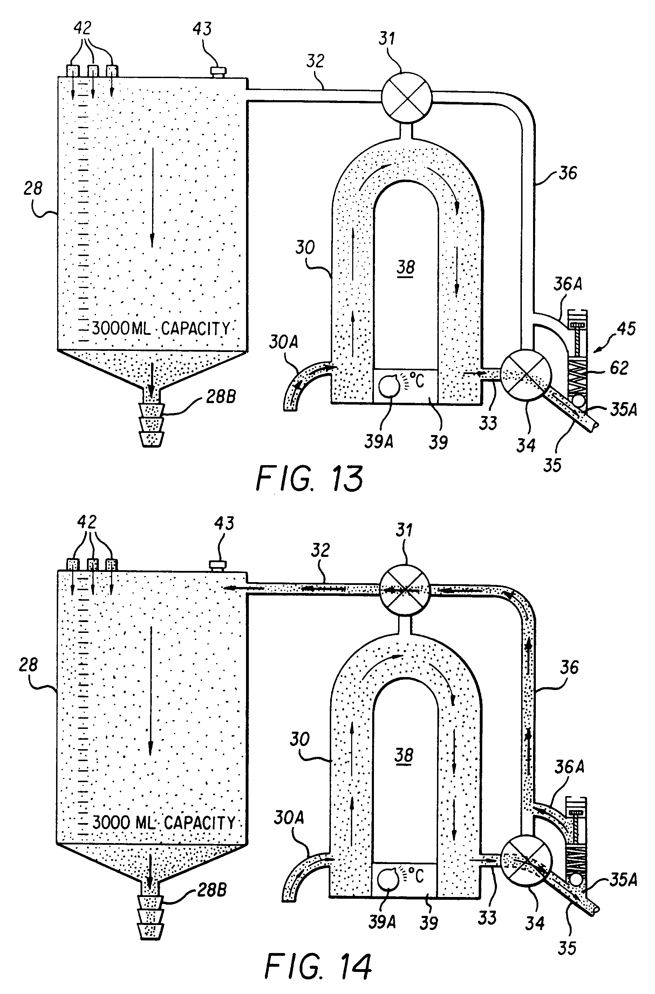

Referring now to Fig. 13, the normal mode of operation of RID 10 may be seen with the path of volume flow to the patient. This mode of operation, as illustrated in Fig. 13, follows the purge procedure and the recirculation procedure for warming volume to the desired temperature. The filter/reservoir 28 is filled from the volume source and flows from outlet port 28B of the filter/reservoir 28 through the pump to the inlet port 30A of heat exchange component 30. The by-pass-to-patient valve 34 is opened to the patient and closed to the purge by-pass line 36 and pressure control valve 45 is also closed due to bias of spring 62 on ball 63, keeping ball 63 on its seat 35A.

Referring now to Fig. 14, there is shown the flow path of volume when there is an increase of back pressure in the volume pathway to the patient. As indicated previously spring loaded pressure control valve 45 is normally closed during all modes of operation. In Fig. 14, normal flow to patient is depicted with pressure control valve 45 open. This valve is a further safety feature and is positioned in the system to sense an over-pressure condition to the patient. When pressure is too great to the patient, i.e., higher than the range set for the valve 45, 230-300 mm Hg, the valve 45 will release back pressure from the patient. When valve 45 is opened, volume will flow into the purge by-pass line 36A and return to the filter/reservoir 28 for recirculation via lines 36 and 32. In this event, the operator would adjust the pressure to the patient by reducing the rpm of pump 22 or reset the spring loaded pressure control valve 45 to a new setting, whichever is indicated. The spring loaded pressure control valve 45 is a safety device which prevents elevated perfusion pressure to the patient.

Having described the various components of the rapid infusion device 10, the set-up and assembly of the RID 10 is as follows: Attach heater element 38 to pump housing 21 through the use of mounting bracket 37 which is attached to pump housing 21 by a first pair of bolts and a second pair of bolts for securing heater element 38 to bracket 37. Next, place the new disposable unit 50 which includes filter/resevoir 28 and heat exchange component 30 with their associated pathways and valves, on top of roller pump aligning the female inverted "U" of heat exchange component 30 with the male inverted "U" of heating element 38. Ensure good surface contact between the walls of these two units. The fit of the units will be sufficient to provide stability for these disposable components when seated in position on roller pump 22. Attach PVC line 29 to outlet port 28B of filter/reservoir 28. Place the other end of PVC line 29 into pump raceway 23 and secure in place, then attach the free end of line 29 to stem portion 30A of heat exchange component 30. Connect power cord 40 of heating element 38 to the auxiliary power outlet in the right side of control panel 12 as indicated by plug 41 in Fig. 1. Attach the catheter to the line to patient line 35 by engaging the male luer lock provided for this purpose.

When the disposable components have been properly placed with respect to the permanent components of RID 10 and the appropriate pathways have been connected as required, the RID 10 is ready for the pre-operating checks which include the following:
1. Check the connections at the filter/reservoir exit port 28B and heater unit inlet port 30A.
2. Rotate purge-by-pass valve 31 and by-pass-to-patient valve 34 to insure freedom of movement in the valves 31 and 34.
3. Check all power connections. Assure proper grounding.
4. Check air vent screw cap 43A for freedom of movement.
5. Check PVC line 29 for proper positioning in track raceway 23, 23A, of pump 22.
6. Check the setting on Spring Loaded Pressure Control valve 45 for a range of 230-300 mm Hg.

After the pre-operating procedural checks have been made and each item is found to be in good working order, a review of the operating modes of RID 10 in the order of undertaking may be seen to include:

### A. System Purge

1. Loosen Air vent cap 43A.
2. Turn Purge-by-pass Valve 31 to the purge position.
3. Turn By-Pass-To-Patient Valve 34 to the closed position.
4. Turn heater unit thermostat 39 to the OFF position.
5. Commence flow of volume to the filter/reservoir 28 from the volume supply source.
6. Allow the filter/reservoir 28 to fill to at least 200 ml or alternative amount to be pumped.
7. Turn the on/off switch 17 to the ON position.
8. Place AC/DC selection switch 18 in the AC position.
9. Place the Stop/Forward switch 19 in the FORWARD position.
10. Leave the RPM control knob 14 in the ZERO setting until flow is desired in the system.
11. Rotate RPM control knob 14 forward to start the rotation of the pump's rollers 26 in the raceway formed by wall 23.
12. Continue this mode of operation until the system is free of air.

### B. By-Pass Mode (Recirculation)

1. This mode of operation normally follows the preceding system purge operation. At this time the volume to be infused fills the system and has been purged of air.
2. The various steps required for this mode are as follows:
   a. Turn the Purge-By-Pass valve 31 to the by-pass position.
   b. Turn the thermostat setting knob 39A to the desired setting.
   c. Turn the By-Pass-To-Patient valve 34 to the by-pass position.
   d. Rotate the RPM knob 14 forward to restart the flow. Continue this mode of operation until the volume has been heated to above 37 degrees C. or a higher alternative temperature.

### C. Volume To Patient (Normal Operation)

1. This mode is considered to be the normal mode of operation. This mode follows the purge and recirculation modes.
2. The various steps required for this mode are as follows:
   a. Attach the catheter line (not shown) to patient.
   b. Check the setting on the Spring Loaded Pressure Control Valve 45.
   c. Open By-Pass-To-Patient Valve 34 to the patient and monitor patient and volume level in the RID 10.

### D. Volume To Patient (Pressure Control Valve Open)

This condition will only occur during the normal volume to patient operation of the RID 10. The Spring Loaded Pressure Control Valve 45 will open allowing the volume to move up the Purge By-Pass line 36 toward the filter/reservoir 28 if the pressure to patient exceeds the predetermined limit. Two steps may be taken to remedy this condition. (1) Reduce the RPM of roller pump 22 by turning RPM knob 14 counterclockwise or adjust Spring-Loaded Pressure Control Valve 45 to a new setting.

Following modes C or D, the disconnect procedure is as follows:
A. Disconnect exit line 35 to patient (DISPOSE).
B. Remove connecting line 29 from filter/resevoir 28 to heater unit 30 (DISPOSE).
C. Turn heat exchange unit thermostat knob 39A to the OFF position.
D. Lift the disposable components, i.e., filter/resevoir 28, heater unit 30, and flow lines 32, 36 off the top of roller pump 22 (DISPOSE).
E. Set aside a new sterile set of disposable parts for the next use of RID 10.
F. Turn all controls to the OFF position and unplug the plug 41.

Roller pump 22 is a standard industrial roller pump commonly found in hospital applications of a various nature, but normally those applications which require the movement of liquids at a modulated rate. Any pump of similar capability may be used in conjunction with the disposable components and other disposable parts of the RID.

While the invention has been described in its preferred embodiment, it is to be understood that the words which have been used are words of description rather than limitation and that changes may be made within the purview of the appended claims without departing from the full scope of the invention.

## Claims

1. A rapid infusion device for the rapid delivery of filtered, bubble free, warmed volume to a patient, comprising a disposable component which includes a reservoir (28) for the volume and all sub-components that come into contact with the volume infused to a patient; a permanent durable unit which comprises the reusable components including drive means operably connected to pump means (22); heater means (38) and associated controls (39) therefor, and a disposable length of flexible tubing (29) which serves as the pumping chamber of said pump means (22) and interconnects pathways (28b, 30a) of said disposable component, which also comprises a heat exchange unit (30) cooperating with the heater (38), whereby a warm, filtered, bubble free volume of blood or other fluid may be rapidly infused into a patient by a single medical person;
characterised in that said disposable component comprises an integral unit (50) including
- said filter/reservoir (28) with filter means (55) mounted therein;
- said heat exchange unit (30) cooperating with the said heater means (38),
- said volume pathways (28a, 30a),
- valve means (31, 34) included by said heat exchange unit (30) and said pathways (28a, 30a),
- said disposable length of tubing, interconnected with said reservoir means (28) and said heat exchange unit (30) at opposite ends thereof;
and in that said filter/reservoir (28), said heat exchange unit (30), said valve means (31, 34) and said volume pathways of said disposable unit (50) are together formed of two halves which are joined and sealed for liquid integrity after placement of said filter means (55) in said reservoir (28).

2. A rapid infusion device according to claim 1, wherein the said filter/reservoir (28) comprises a plurality of supply inlets (42), a return port (32A), an outlet (28B) and an air purge vent (43) for removing air from the system prior to infusing the pumped volume to a patient, said reservoir (28) further including baffle means (46) which is positioned below said plurality of supply inlets (42) and said return port (32A) and filter means (55) mounted within said reservoir.

3. A rapid infusion device according to claim 2, wherein said baffle means comprises an arcuate baffle (46) which directs in coming volume into a horizontal direction toward and through said filter means (55) whereby a vortex action is created in the flow and serves to prevent possible damage to the volume by impact.

4. A rapid infusion device according to claim 2 or claim 3, wherein said filter means comprises a two stage filter with the first stage (57) serving to remove particles in excess of 150 microns from said volume and said second stage is a multi-layered wafer assembly (56) of filtering material which removes any impurities of 27-40 microns in size.

5. A rapid infusion device according to claim 4, wherein said first stage filter (57) is a coarse sponge plastic material positioned on the fill side of said reservoir (28).

6. A rapid infusion device according to claim 4 or claim 5, wherein said multi-layered wafer assembly comprises three layers of filtering material; the first layer (58) consisting of nylon mesh, followed by a layer (59) of Cottonow "3" material which is sandwiched by a third layer which is another layer of nylon mesh with both stages of said filter means being supported by a filter frame (56) within said reservoir (28).

7. A rapid infusion device according to anyone of claims 1 to 6, wherein all subcomponents that will come into contact with the volume infused to the patient, including the disposable length of tubing (29) serving as a pumping chamber, are contained in the pre-assembled unitary disposable unit (50) which is supported by housing means (21), which also supports said pump means (22), said drive means, said heater means (38) and said related control means (39).

8. A rapid infusion system according to claim 7, wherein a permanent reusable subsystem comprises motor drive means and second housing means (11) enclosing said motor drive means (24) and including control panel means (12); said first housing means (21) mounted on top of said second housing means (11); said pump means (22) and said heater means (38) mounted on said first housing means (21); said first housing means (21) serving as a supporting base for said preassembled unitary disposable unit (50).

9. A rapid infusion system according to claim 7 or claim 8, wherein said filter/reservoir (28) includes said air vent (43) mounted on its uppermost wall, a transverse baffle (46) mounted on a rear tell immediately below said plurality of inlets (42); said filter/reservoir (28) having a conical bottom wall (28A) with an outlet port (28B) positioned at the lowermost point of said conical bottom wall (28A) and said conical wall further including a pair of flanges (47, 48) projecting upwardly from the interior surface of one side of said conical wall (28A).

10. A rapid infusion system according to any one of claims 7 to 9, wherein said heat exchange unit (30) comprises an inverted U/shaped member (30) having an inlet (30A) and a pair of outlets (31A, 33), the exterior wall, forming the inner wall (51) of said U-shaped member (30) , provided with a coating of aluminum-telfa to improve the heat transfer rate when placed in contact with the outer wall (38A) of said heater means (38).

11. A rapid infusion system according to any one of claims 7 to 10, wherein said volume pathways comprise connecting lines (33, 36) from said heat exchange unit (30) to said valve means (31, 34) directing volume to either said reservoir (28) or said patient.

12. A rapid infusion system according to any one of claims 7 to 11, wherein said valve means includes a pair of two-way valves (31, 34) and a spring loaded system pressure control valve (45), said spring loaded system pressure control valve (45) being adjustable to a predetermined pressure; said pair of two-way valves (31, 34) being manually operable to direct volume flow in the desired direction whereas said spring loaded system pressure control valve (45) automatically opens to by-pass volume back to said reservoir (28) when said predetermined pressure setting has been exceeded.

13. A rapid infusion system according to claim 12, wherein said spring loaded system pressure control valve (45) comprises a cylindrical housing (66) including a stationary base (65) therein, said stationary base (65 having a central threaded aperture therein, an adjusting screw (60) having a head portion (67) and a threaded shank portion of lesser diameter as said threaded shank portion being received in said central threaded aperture of said stationary base (65), perforated disc means (61) attached to the lowermost end of said threaded shank; a coil spring (62) positioned below said perforated disc (61) with its uppermost end engaging said perforated disc (61) and its lowermost end engaging a ball (63); a seat (35A) formed below said ball (63) with said coil spring (62) urging said ball (63) into sealing engagement with said seat (35A), the upper portion of said cylindrical housing (66) fluidly connected with a return line (36A) to said reservoir (28) whereby when said predetermined pressure setting of said adjusting screw (60) is exceeded said ball (63) is raised from said seat (35A) permitting volume to flow up through said cylindrical housing (66) to said return line (36A) permitting a portion of said volume to be returned to said reservoir (28).

14. A rapid infusion system according to claim 13, wherein said head portion (67) of said adjusting screw is provided with a slot therein for receiving a screwdriver and the bias of said coil spring (62) may be increased or decreased as desired.

15. A rapid infusion system according to any one of claims 7 to 14, wherein said motor drive means is a reversible, variable speed alternating current motor.

16. A rapid infusion system according to any of claims 7 to 15, wherein said control panel means (12) includes a tachometer (13) and revolution control (14) for said motor drive means, an AC/DC switch (18) , an ON/OFF switch (17), a FORWARD/REVERSE switch (19) and AC/DC fuses (15, 16).

17. A rapid infusion system according to any one of claims 7 to 16, wherein said pump means comprises a roller pump (22) having a shaft (24) driven by said motor drive means, a pair of arms (25) operably connected to said shaft (24) and extending in opposite directions therefrom; each of said pair of arms (25) having a roller (26) mounted thereon whereby rotation of said shaft (24) rotates said arms (25) and said rollers (26) sequentially come into contact with said disposable lenght of tubing (29) interconnecting said reservoir (28) and said heat exchange unit (30) to provide a pumping action to said volume within said disposable length of tubing (29).

18. A rapid infusion system according to any one of claims 7 to 17, wherein said heater means comprises a heater element (38) having an outer surface (51) forming an inverted U-shape and a thermostat (39) wich controls the temperature of said heater element (38).

## Patentansprüche

1. Schnellinfusionsvorrichtung für die Schnellzufuhr eines gefilterten, blasenfreien, erwärmten Volumens zu einem Patienten, mit einer austauschbaren Komponente, die ein Reservoir (28) für das Volumen und sämtliche Unterkomponenten beinhaltet, die mit dem einem Patienten infundierten Volumen in Berührung kommen, einer bleibenden Dauereinheit, die die wiederverwendbaren Komponenten aufweist, einschließlich eines Antriebsmittels, das mit einer Pumpeneinrichtung (22) wirkungsmäßig verbunden ist, einer Heizeinrichtung (38) und ihr zugehöriger Steuerung und einer austauschbaren Länge einer flexiblen Rohrleitung (29), welche als Pumpenkammer der genannten Pumpeneinrichtung (22) dient und Durchgänge (28b, 30a) der genannten austauschbaren Komponente verbindet, welche auch eine Wärmetauschereinheit (30) aufweist, die mit der Heizeinrichtung (38) zusammenwirkt, wodurch ein warmes, gefiltertes, blasenfreies Volumen aus Blut oder einer anderen Flüssigkeit durch eine einzige medizinische Person schnell einem Patienten infundiert werden kann, dadurch gekennzeichnet, daß die genannte austauschbare Komponente eine integrale Einheit (50) aufweist, beinhaltend
- das genannte Filter/Reservoir (28) mit darin angeordneter Filtereinrichtung (55);
- die genannte Wärmetauschereinheit (30), die mit der genannten Heizeinrichtung (38) zusammenwirkt;
- die genannten Durchgänge (28b, 30a) für das Volumen;
- eine Ventileinrichtung (31, 34), die der genannten Wärmetauschereinheit (30) und den genannten Durchgängen (28b, 30a) zugehörig ist;
- die genannte austauschbare Länge der Rohrleitung, die mit dem genannten Reservoir (28) und der genannten Wärmetauschereinheit (30) an ihren beiden entgegengesetzten Enden verbunden ist;
und dadurch, daß das genannte Filter/Reservoir (28), die genannte Wärmetauschereinheit (30), die genannte Ventileinrichtung (31, 34) und die genannen Volumendurchgänge der genannten austauschbaren Einheit (50) zusammen aus zwei Hälften gebildet sind, die nach in Stellung bringen der genannten Filtereinrichtung (55) in dem genannten Reservoir (28) miteinander verbunden und flüssigkeitssicher abgedichtet sind.

2. Schnellinfusionsvorrichtung nach Anspruch 1, bei der das genannte Filter/Reservoir (28) eine Mehrzahl Versorgungseinlässe (42), einen Rücklaufeinlaß (32A), einen Auslaß (28B) sowie ein Entlüftungsventil (43) zum Entfernen von Luft aus dem System vor dem Infundieren des gepumpten Volumens in einen Patienten aufweist, wobei das genannte Reservoir (28) außerdem eine Leitflächeneinrichtung (46) beinhaltet, die unterhalb der genannten Mehrzahl der Versorgungseinlässe (42) angeordnet ist, und der genannte Rücklaufeinlaß (32A) und die Filtereinrichtung (55) innerhalb des genannten Reservoirs angeordnet sind.

3. Schnellinfusionsvorrichtung nach Anspruch 2, bei der die genannte Leitflächeneinrichtung eine bogenförmige Leitwand (46) aufweist, die das ankommende Volumen in eine horizontale Richtung gegen die genannte Filtereinrichtung (55) hin und durch diese hindurch lenkt, wodurch eine Vewirbelung in der Strömung erzeugt wird, was dazu dient, eine mögliche Beschädigung des Volumens durch Prallwirkung zu verhindern.

4. Schnellinfusionsvorrichtung nach Anspruch 2 oder Anspruch 3, bei der die genannte Filtereinrichtung ein Zweistufenfilter aufweist, wobei die erste Stufe (57) dazu dient, Teilchen größer als 150 Mikron aus dem genannten Volumen zu entfernen und die genannte zweite Stufe ein mehrlagiger Sandwichaufbau (56) aus Filtermaterial ist, das jedwede Verunreinigungen von 27-40 Mikron Größe entfernt.

5. Schnellinfusionsvorrichtung nach Anspruch 4, bei der die genannte erste Filterstufe (57) ein grobes Schwammaterial aus Kunststoff ist, das an der Füllseite des genannten Reservoirs (28) angeordnet ist.

6. Schnellinfusionsvorrichtung nach Anspruch 4 oder Anspruch 5, bei der der genannte, mehrlagige Sandwichaufbau drei Lagen von Filtermaterial aufweist, wobei die erste Lage (58) aus Nylonnetz besteht, gefolgt durch eine Lage (59) aus Cottonow "3"-Material, welche neben einer dritten Lage gelegen ist, die eine weitere Lage aus Nylonnetz ist, wobei beide Stufen der genannten Filtereinrichtung durch einen Filterrahmen (56) innerhalb des genannten Reservoirs (28) getragen sind.

7. Schnellinfusionsvorrichtung nach einem der Ansprüche 1 bis 6, bei der sämtliche Unterkomponenten, die mit dem dem Patienten infundierten Volumen in Berührung kommen, einschließlich der austauschbaren Länge der Leitung (26), welche als Pumpenkammer dient, in der vorgefertigten, einheitlichen, austauschbaren Einheit (50) enthalten sind, die durch eine Gehäuseeinrichtung (21) getragen ist, welche auch die genannte Pumpeneinrichtung (22), die genannte Antriebseinrichtung, die genannte Heizeinrichtung (38) und die genannte zugehörige Steuereinrichtung (39) trägt.

8. Schnellinfusionsvorrichtung nach Anspruch 7, bei der ein bleibendes, wiederverwendbares Untersystem eine Antriebsmotoreinrichtung und eine zweite Gehäuseeinrichtung (11) aufweist, die die genannte Antriebsmotoreinrichtung (24) einschließt und eine Steuerpulteinrichtung (12) beinhaltet, die genannte erste Gehäuseinrichtung (21) an der Oberseite der genannten zweiten Gehäuseeinrichtung (11) angeordnet ist, die genannte Pumpeneinrichtung (22) und die genannte Heizeinrichtung (38) an der genannten ersten Gehäuseeinrichtung (21) angeordnet sind, wobei die genannte erste Gehäuseeinrichtung (21) als tragende Basis für die genannte vorgefertigte, einheitliche, austauschbare Einheit (50) dient.

9. Schnellinfusionsvorrichtung nach Anspruch 7 oder Anspruch 8, bei der das genannte Filter/Reservoir (28) das genannte Entlüftungsventil (43), an seiner obersten Wand angeordnet, beinhaltet, eine quer verlaufende Leitwand (46) unmittelbar unter der genannten Mehrzahl von Einlässen angeordnet ist, das genannte Filter/Reservoir (28) eine konische Bodenwand (28A) mit einem Auslaß (28B) aufweist, der an der untersten Stelle der genannten konischen Bodenwand (28A) gelegen ist, und die genannte konische Wand außerdem ein Paar Leisten (47, 48) beinhaltet, die von der Innenfläche einer Seite der genannten konischen Wand (28A) nach oben vorspringen.

10. Schnellinfusionsvorrichtung nach einem der Ansprüche 7 bis 9, bei der die genannte Wärmetauschereinheit (30) ein als umgekehrtes U geformtes Glied (30) aufweist, das einen Einlaß (30A) und ein Paar Auslässe (31A, 33) aufweist, wobei die Außenwand, die die Innenwand (51) des genannten U-förmigen Gliedes (30) bildet, mit einer Beschichtung aus Aluminium-Telfa versehen ist, um die Wärmeübergangsrate zu verbessern, wenn sie in Anlage an die äußere Wand (38A) der genannten Heizeinrichtung (38) gebracht ist.

11. Schnellinfusionsvorrichtung nach einem der Ansprüche 7 bis 10, bei der die genannten Durchgänge für das Volumen Verbindungsleitungen (33, 36) von der genannten Wärmetauschereinheit (30) zu den genannten Ventileinrichtungen (31, 34) beinhalten, um Volumen entweder zu dem genannten Reservoir (28) oder dem genannten Patienten zuzuführen.

12. Schnellinfusionsvorrichtung nach einem der Ansprüche 7 bis 11, bei der die genannte Ventileinrichtung ein Paar Zweiwegventile (31, 34) und ein federbelastetes Steuerventil (45) für den Systemdruck beinhaltet, wobei das genannte federbelastete Steuerventil (45) für den Systemdruck auf einen vorbestimmten Druck einstellbar ist, das genannte Paar Zweiwegventile (31, 34) manuell betätigbar ist, um den Volumenstrom in die gewünschte Richtung zu lenken, während das genannte federbelastete Steuerventil (45) für den Systemdruck selbsttätig öffnet, um einen Bypass für Volumen in das genannte Reservoir (28) zurück herzustellen, wenn die genannte vorbestimmte Druckeinstellung überschritten ist.

13. Schnellinfusionsvorrichtung nach Anspruch 12, bei der das genannte federbelastete Steuerventil (45) für den Systemdruck ein zylindrisches Gehäuse (66) mit einem in ihm befindlichen stationären Grundteil (65) aufweist, wobei der genannte stationäre Grundteil (65) eine zentrale Gewindebohrung in sich aufweist, eine Einstellschraube (60) mit einem Kopfteil (67) und einem Gewindeschaftteil kleineren Durchmessers in der genannten zentralen Gewindebohrung des stationären Grundteils (65) aufgenommen ist, eine perforierte Scheibe (61) am untersten Ende des genannten Gewindeschaftes angebracht ist, eine Schraubenfeder (62) unter der genannten perforierten Scheibe (61) so angeordnet ist, daß sie mit ihren oberen Ende die perforierte Scheibe (61) berührt und ihr unteres Ende an einer Kugel (63) anliegt, ein Sitz (35A) unterhalb der genannten Kugel (63) ausgebildet ist, wobei die genannte Schraubenfeder (62) die genannte Kugel (63) in abdichtende Anlage an den genannten Sitz (35A) drängt, der obere Teil des genannten zylindrischen Gehäuses (66) in Fluidverbindung mit einer Rücklaufleitung (36A) zum genannten Reservoir (28) ist, wodurch, wenn die genannte vorbestimmte Druckeinstellung der genannten Einstellschraube (60) überschritten ist, die genannte Kugel (63) von dem genannten Sitz (35A) abgehoben wird, um zu ermöglichen, daß Volumen nach oben durch das genannte zylindrische Gehäuse (66) hindurch zu der genannten Rücklaufleitung (36A) strömt, um zu ermöglichen, daß ein Teil des genannten Volumens in das genannte Reservoir (28) zurückgeführt wird.

14. Schnellinfusionvorrichtung nach Anspruch 13, bei der der genannte Kopfteil (67) der genannten Einstellschraube mit einem in ihm befindlichen Schlitz versehen ist, so daß ein Schraubendreher aufnehmbar ist und die Vorspannung der genannten Schraubenfeder (62) nach Wunsch erhöht oder verringert werden kann.

15. Schnellinfusionsvorrichtung nach einem der Ansprüche 7 bis 14, bei der die genannte Antriebsmotoreinrichtung ein umsteuerbarer Wechselstrommotor veränderlicher Drehzahl ist.

16. Schnellinfusionsvorrichtung nach einem der Ansprüche 7 bis 15, bei der die genannte Steuerpulteinrichtung (12) ein Tachometer (13) und eine Drehzahlsteuerung (14) für die genannte Antriebsmotoreinrichtung, einen Wechselstrom/Gleichstromschalter (18), einen Ein/Ausschalter (17), einen Vorwärts/Rückwärtsschalter (19) sowie Wechselstrom/Gleichstromsicherungen (15, 16) beinhaltet.

17. Schnellinfusionsvorrichtung nach einem der Ansprüche 7 bis 16, bei der die genannte Pumpeneinrichtung eine Walzenpumpe (22) beinhaltet, die eine Welle (24), welche durch die genannte Antriebsmotoreinrichtung angetrieben ist, und ein Paar Arme (25), die wirkungsmäßig mit der genannten Welle (24) verbunden sind und sich von dieser in zueinander entgegengesetzte Richtungen erstrecken, beinhaltet, wobei jeder Arm (25) des genannten Paares eine an ihm angeordnete Walze (26) aufweist, wobei die Drehung der genannten Welle (24) die genannten Arme (25) und die genannten Walzen (26) nacheinander in Berührung mit der genannten austauschbaren Länge der Rohrleitung (29) bringt, die das genannte Reservoir (28) und die genannte Wärmetauschereinheit (30) verbindet, um eine Pumpwirkung für das genannte Volumen innerhalb der genannten austauschbaren Länge der Leitung (29) zur Verfügung zu stellen.

18. Schnellinfusionsvorrichtung nach einem der Ansprüche 7 bis 17, bei der die genannte Heizeinrichtung ein Heizelement (28), das eine äußere Oberfläche (51) besitzt, die eine umgekehrte U-Form bildet, sowie einen Thermostat (39) beinhaltet, der die Temperatur des genannten Heizelements (38) steuert.

## Revendications

1. Dispositif de perfusion rapide pour administrer rapidement à un patient un volume chauffé, filtré, dépourvu de bulles, comprenant un composant jetable qui comprend lui-même un réservoir (28) pour contenir le volume et tous les sous-composants qui entrent en contact avec le volume perfusé à un patient ; une unité durable permanente, qui comprend les composants réutilisables, y compris les moyens d'entraînement reliés fonctionnellement à des moyens formant pompe (22) ; des moyens chauffants (38) et des commandes (39) correspondantes pour ces moyens, et une longueur jetable de tube souple (29) qui joue le rôle de la chambre de pompage desdits moyens formant pompe (22) et qui interconnectent des trajets d'écoulement (28b, 30a) dudit composant jetable, qui comprend en outre une unité d'échange de chaleur (30) coopérant avec l'élément chauffant (38), de sorte qu'un volume de sang ou d'un autre fluide, chaud, filtré, exempt de bulles peut être rapidement perfusé à un patient par un membre du personnel médical opérant seul ;
caractérisé en ce que ledit composant jetable comprend une unité d'une seule pièce (50) comprenant elle-même :
- ledit filtre/réservoir (28) muni de moyens formant filtre (55) montés à l'intérieur ;
- ladite unité d'échange de chaleur (30) coopérant avec lesdits moyens chauffants (38),
- lesdits trajets (28a, 30a) d'écoulement du volume,
- des moyens formant soupapes (31, 34) inclus dans ladite unité d'échange de chaleur (30) et dans lesdits trajets d'écoulement (28a, 30a),
- ladite longueur jetable de tube, qui est interconnectée auxdits moyens formant réservoir (28) et à ladite unité d'échange de chaleur (30) à ses extrémités opposées ;
et en ce que ledit filtre/réservoir (28), ladite unité d'échange de chaleur (30), lesdits moyens formant soupapes (31, 34) et lesdits trajets d'écoulement du volume de ladite unité jetable (50) sont formés ensemble de deux moitiés qui sont réunies et raccordées à joint étanche pour l'intégrité du liquide après la mise en place desdits moyens formant filtre (55) dans ledit réservoir (28).

2. Dispositif de perfusion rapide selon la revendication 1, dans lequel ledit filtre/réservoir (28) comprend une pluralité d'entrées d'alimentation (42), un orifice de retour (32A), une sortie (28B) et un évent de purge d'air (43) servant à éliminer l'air du système avant de perfuser le volume pompé à un patient, ledit réservoir (28) comprenant en outre des moyens formant chicane (46) qui sont positionnés au-dessous de ladite pluralité d'entrées d'alimentation (42) et dudit orifice de retour (32A) et des moyens formant filtre (55) montés dans ledit réservoir.

3. Dispositif de perfusion rapide selon la revendication 2, dans lequel lesdits moyens formant chicane comprennent une chicane (46) de forme courbe qui dirige le volume entrant dans une direction horizontale vers et à travers lesdits moyens formant filtre (55), de sorte qu'il se crée une action de vortex dans l'écoulement et qu'elle sert à éviter toute détérioration éventuelle du volume par impact.

4. Dispositif de perfusion rapide selon la revendication 2 ou la revendication 3, dans lequel lesdits moyens formant filtre comprennent un filtre à deux étages, le premier étage (57) servant à éliminer les particules de plus de 150 microns dudit volume et le deuxième étage est un ensemble de disques multicouche (56) de matière filtrante qui élimine les impuretés d'une dimension de 27 à 40 microns.

5. Dispositif de perfusion rapide selon la revendication 4, dans lequel ledit premier étage (57) du filtre est une matière plastique spongieuse à grands pores positionnée sur le côté de remplissage dudit réservoir (28).

6. Dispositif de perfusion rapide selon la revendication 4 ou la revendication 5, dans lequel ledit ensemble de disques multicouche comprend trois couches de matière filtrante ; la première couche (58) étant composée d'un tamis de nylon et étant suivie d'une couche (59) de matière Cottonow "3" qui est recouverte d'une troisième couche, qui est une autre couche de tamis de nylon, les deux étages desdits moyens formant filtre étant supportés à l'intérieur dudit réservoir (28) par un cadre (56) de filtre.

7. Dispositif de perfusion rapide selon une quelconque des revendications 1 à 6, dans lequel tous les sous-composants qui entrent en contact avec le volume perfusé au patient, y compris la longueur jetable de tube (29) qui sert de chambre de pompage, sont contenus dans l'unité jetable unitaire préassemblée (50) qui est supportée par des moyens formant boîtier (21) qui supportent aussi lesdits moyens formant pompe (22), lesdits moyens d'entraînement, lesdits moyens chauffants (38) et lesdits moyens de commande correspondants (39).

8. Dispositif de perfusion rapide selon la revendication 7, dans lequel un sous-système réutilisable permanent comprend des moyens d'entraînement à moteur et des deuxièmes moyens (11) formant boîtier qui renferment lesdits moyens d'entraînement à moteur (24) et comprennent des moyens (12) formant panneau de commande ; lesdits premiers moyens (21) formant boîtier montés sur lesdits deuxièmes moyens (11) formant boîtier ; lesdits moyens formant pompe (22) et lesdits moyens chauffants (38) montés sur lesdits premiers moyens (21) formant boîtier ; lesdits premiers moyens (21) formant boîtier servant de base de support pour ladite unité jetable unitaire (50) préassemblée.

9. Dispositif de perfusion rapide selon la revendication 7 ou la revendication 8, dans lequel ledit filtre/réservoir (28) comprend ledit évent d'air (43) monté sur sa paroi extrême supérieure, une chicane transversale (46) montée sur une paroi arrière immédiatement au-dessous de ladite pluralité d'entrées (42), ledit filtre/réservoir (28) ayant une paroi de fond conique (28A) munie d'un orifice de sortie (28B) positionné au point extrême inférieur de ladite paroi de fond conique (28A) et ladite paroi conique comprenant en outre une paire de nervures (47, 48) qui font saillie vers le haut sur la surface intérieure d'une face de ladite paroi conique (28A).

10. Dispositif de perfusion rapide selon une quelconque des revendications 7 à 9, dans lequel ladite unité d'échange de chaleur (30) comprend un élément (30) en forme de U inversé ayant une entrée (30A) et une paire de sorties (31A, 33), la paroi extérieure qui forme la paroi intérieure (51) dudit élément (30) en forme de U, munie d'un revêtement d'aluminium-telfa, pour améliorer le taux de transmission de chaleur lorsqu'elle est placée en contact avec la paroi extérieure (38A) desdits moyens chauffants (38).

11. Système de perfusion rapide selon une quelconque des revendications 1 à 10, dans lequel lesdits trajets d'écoulement du volume comprennent des lignes de raccordement (33, 36) allant de ladite unité d'échange de chaleur (30) auxdits moyens formant soupapes (31, 34) qui dirigent le volume, soit vers ledit réservoir (28), soit vers ledit patient.

12. Système de perfusion rapide selon une quelconque des revendications 7 à 11, dans lequel lesdits moyens formant soupapes comprennent une paire de soupapes à deux voies (31, 34) et une soupape (45) de commande de la pression du système, chargée par ressort, ladite soupape (45) de commande de la pression du système chargée par ressort pouvant être ajustée sur une pression déterminée, ladite paire de soupapes à deux voies (31, 34) pouvant être manoeuvrées manuellement pour diriger le volume d'écoulement dans la direction désirée, tandis que ladite soupape (45) de commande de la pression du système chargée par ressort s'ouvre automatiquement pour renvoyer le volume dérivé audit réservoir (28) lorsque la valeur fixée de réglage de pression prédéterminée a été dépassée.

13. Système de perfusion rapide selon la revendication 12, dans lequel ladite soupape (45) de commande de la pression du système chargée par ressort comprend un boîtier cylindrique (66) renfermant intérieurement une base fixe (65), ladite base fixe (65) ayant intérieurement une ouverture centrale filetée, une vis de réglage (60) ayant une partie tête (67) et une partie queue filetée de plus petit diamètre, ladite partie queue filetée étant logée dans ladite ouverture centrale filetée de ladite base fixe (65), des moyens formant disque perforé (61) fixés à l'extrémité extrême inférieure de ladite tige filetée ; un ressort hélicoïdal (62) positionné au-dessous dudit disque perforé (61) avec son extrémité extrême supérieure appuyée contre ledit disque perforé (61) et son extrémité extrême inférieure appuyée contre une bille (63) ; un logement (35A) formé au-dessous de ladite bille (63), ledit ressort hélicoïdal (62) appliquant ladite bille (63) en contact d'étanchéité avec ledit siège (35A), la partie supérieure dudit boîtier cylindrique (66) étant reliée fluidiquement à une ligne de retour (36A) amenant audit réservoir (28), de sorte que, lorsque ladite valeur fixée de pression prédéterminée de ladite vis de réglage (60) est dépassée, ladite bille (63) est soulevée dudit siège (35A) en permettant au volume de remonter à travers ledit boîtier cylindrique (66) jusqu'à ladite ligne de retour (36A) en permettant à une partie dudit volume d'être renvoyée audit réservoir (28).

14. Système de perfusion rapide selon la revendication 13, dans lequel ladite partie tête (67) de ladite vis de réglage est munie d'une fente pour recevoir un tournevis et la sollicitation dudit ressort hélicoïdal (62) peut être augmentée ou réduite selon le besoin.

15. Système de perfusion rapide selon une quelconque des revendications 7 à 14, dans lequel lesdits moyens d'entraînement à ressort sont constitués par un moteur réversible, à courant alternatif et à vitesse variable.

16. Système de perfusion rapide selon une quelconque des revendications 7 à 15, dans lequel lesdits moyens (12) formant panneau de commande comprennent un tachymètre (13) et une commande de vitesse (14) pour lesdits moyens d'entraînement à moteur, un commutateur CA/CC (18), un commutateur marche/arrêt (17), un commutateur marche avant/marche arrière (19) et des fusibles CA/CC (15, 16).

17. Système de perfusion rapide selon une quelconque des revendications 7 à 16, dans lequel les moyens formant pompe comprennent une pompe à rouleaux (22), ayant un arbre (24) entraîné par lesdits moyens d'entraînement à moteur, deux bras (25) connectés fonctionnellement audit arbre et qui partent de cet arbre dans des directions opposées ; chacun desdits bras (25) de la paire portant un galet (26) monté sur lui, de sorte que la rotation dudit arbre (24) fait tourner lesdits bras (25) et lesdits galets (26) entrent successivement en contact avec ladite longueur de tube jetable (29) qui interconnecte ledit réservoir (28) à ladite unité d'échange de chaleur (30) pour exercer une action de pompage sur ledit volume contenu dans ladite longueur de tube jetable (29).

18. Système de perfusion rapide selon une quelconque des revendications 7 à 17, dans lequel lesdits moyens chauffants comprennent un élément chauffant (38) ayant une surface extérieure (51) en forme de U inversé et un thermostat (39) qui commande la température dudit élément chauffant (38).
